# EUROPEAN PATENT SPECIFICATION

(11) **EP 4 649 123 B1**
(45) Date of publication and mention of the grant of the patent: **03.06.2026**
(21) Application number: 24700701.6
(22) Date of filing: 09.01.2024
(51) Int. Cl.: C10G 2/00, C10G 29/20, C07C 1/12, C07C 1/20, C07C 15/04, C07C 15/06, C07C 15/08, C07C 2/00, C10G 50/00, C10G 45/44, C10G 9/36, B01J 21/00, B01J 29/04, C25B 1/04, C07C 2/66, C07C 29/151, B01J 21/06, B01J 23/00, B01J 23/08, B01J 29/06, B01J 35/00

(54) **METHOD FOR PRODUCING FUEL VIA LOWER AROMATICS**
VERFAHREN ZUR HERSTELLUNG VON BRENNSTOFF ÜBER NIEDRIGE AROMATEN
PROCÉDÉ DE PRODUCTION DE CARBURANT PAR L'INTERMÉDIAIRE D'AROMATIQUES INFÉRIEURS

(30) Priority: 10.01.2023 EP 23151002
(43) Date of publication of application: 19.11.2025
(73) Proprietor: TotalEnergies OneTech, 92400 Courbevoie (FR)
(72) Inventor: STEWART, Joseph, 7181 Seneffe (BE); VERMEIREN, Walter, 3530 Houthalen (BE)
(74) Representative: Mellet, Valérie Martine
(86) International application number: PCT/EP2024/050333
(87) International publication number: WO 2024/149723

(56) References cited:
- US-A- 6 059 962
- US-A1- 2008 223 753
- US-A1- 2010 108 568
- YANG WANG ET AL: "Direct and Oriented Conversion of CO2 into Value-Added Aromatics", CHEMISTRY - A EUROPEAN JOURNAL, JOHN WILEY & SONS, INC, DE, vol. 25, no. 20, 19 March 2019 (2019-03-19), pages 5149 - 5153, XP071847893, ISSN: 0947-6539, DOI: 10.1002/CHEM.201806165

## Description

### Field of the invention

The invention relates to methods for producing fuel and to uses thereof.

### Background of the invention

Climate change and the on-going energy transition makes it mandatory to replace fossil-based energy sources. In this context various aspects will be important for society to reach Net Zero by 2050 as currently desired. For example, the valorisation of alternative feedstocks is expected to contribute towards the circular economy and/or reduce the carbon dioxide (CO₂) footprint associated with the final product. However, while many alternative fuels and chemicals are sort after, drop in solutions from alternative feedstocks would allow for existing infrastructure to be maintained.

In this context even the valorisation of CO₂ as a feedstock is considered, sourced from flue gas, bio sources and even direct air capture. Upgrading of CO₂ to chemicals such as methanol has already received some attention to yield sustainable fuel for various applications. However, some applications regularly lead to enhanced requirements for the sustainable fuel. For example, aviation fuel, i.e., fuel to power aircraft, regularly requires to contain larger hydrocarbons and aromatics. This is because larger hydrocarbons and aromatics regularly improve the cold flow properties of the aviation fuel. Such improved cold flow properties prevent fuel freezing at low temperatures of for example -40°C, which are typical for cruising altitudes of the powered aircrafts. In attempts to upgrade CO₂, larger hydrocarbons and aromatics are however regularly more difficult to achieve.

In a different approach, bio conversion and syngas upgrading via Fischer-Tropsch (FT) are also considered as an alternative to a fossil fuel feedstock. Such routes may appear attractive for diesel and gasoline production. However, they are not suitable for 100% aviation fuel, as they crucially lack aromatic content and hence are applied as a blend.

Thus far, there are no established routes of producing a 100% sustainable aviation fuel by bio conversion or syngas upgrading via FT.

Further in the search for sustainable fuel and in particular in the search for aviation fuel, hydrodeoxygenation of fatty acids/triglycerides, CO₂- or bio-sourced syngas upgrading, or bio-olefin oligomerisation have been researched at various Technology Readiness Levels (TRLs). However, for each of these routes, the major product is regularly long hydrocarbon chains and is typically only suitable for up to 50% blend to meet requirements for aviation fuel. This is because these routes do regularly not yield aromatics which are however also required to improve the cold flow properties of aviation fuel.

Overall, there remains a general desire for an improved method for producing fuel and in particular aviation fuel.

YANG WANG ET AL: "Direct and Oriented Conversion of CO2 into Value-Added Aromatics",CHEMISTRY - A EUROPEAN JOURNAL, JOHN WILEY & SONS, INC, DE, vol. 25, no. 20, 19 March 2019 (2019-03-19), pages 5149-5153 discloses a method for producing fuel.

### Problem underlying the invention

It is an object of the present invention to provide a method for producing fuel which at least partially overcomes the drawbacks encountered in the art.

It is in particular an object of the present invention to provide a method for producing fuel which reduces the CO₂ footprint associated with the produced fuel.

It is furthermore an object of the present invention to provide a method for producing fuel which allows for existing infrastructure to be maintained.

It is moreover an object of the present invention to provide a method for producing fuel which has an improved energy efficiency and/or an improved cost efficiency.

It is in particular an object of the present invention to provide a method for producing fuel which meets the requirements for aviation fuel.

### Disclosure of the invention

Surprisingly, it has been found that the problem underlying the invention is overcome by methods according to the claims. Further embodiments of the invention are outlined throughout the description.

The method of the invention is defined in the appended claims.

Logically, steps (i) and (ii) are carried out in the given order, i.e., first step (i) and then step (ii). However, additional steps before or after each of steps (i) and (ii) may also be comprised by the method according to the present invention. The method for producing fuel which comprises C8+ aromatics and C8+ hydrocarbons according to the present invention is herein sometimes also simply referred to as the method for producing fuel according to the present invention.

In step (i), carbon dioxide (CO₂) is at least partially, and preferably completely, converted into methanol (CH₃OH; or MeOH) and carbon monoxide (CO). The conversion occurs in the presence of a metal oxide-based catalyst. As used herein, metal oxide-based means that the catalyst comprises at least one metal oxide, wherein the metal oxide comprises at least one metal cation and an oxide anion. The metal oxide-based catalyst promotes a methanol-forming reaction using CO₂ as a raw material. In other words, the metal oxide-based catalyst lowers the activation energy of a respective methanol-forming reaction. As used herein, a methanol-forming reaction is a reaction which comprises a reaction of CO₂ with hydrogen (H₂) to yield CH₃OH. Such a methanol-forming reaction occurs in step (i) which thus comprises the following reaction: CO₂ + 3H₂ → CH₃OH + H₂O

The above reaction may occur directly, or may be the sum of two partial reactions, i.e., of a first partial reaction: CO₂ + H₂ → CO + H₂O, and
a second partial reaction: CO + 2H₂ → CH₃OH

The metal oxide-based catalyst can be used as such, or in supported form, or in admixture with other solids, for example in an admixture with a zeolite.

As used herein, aromatics are aromatic in the sense of the IUPAC Gold Book. More specifically, aromatics are cyclically conjugated molecular entities with a stability (due to delocalization) significantly greater than that of a hypothetical localized structure (e.g., Kekulé structure). Such cyclically conjugated molecular entities have aromaticity. A method for determining aromaticity can in particular be the observation of diatropicity in the ¹H-NMR spectrum.

As used herein, C6+ aromatics are aromatics which contain 6 or more carbons (or carbon atoms). As used herein, saturated hydrocarbons (herein sometimes also referred to as paraffins) are regularly linear, branched or cyclic alkyls. As used herein unsaturated hydrocarbons are regularly linear, branched or cyclic alkenyls or alkynyls (alkenyls are herein sometimes also referred to as olefins). Saturated and unsaturated hydrocarbons may comprise heteroatoms like oxygen (O), sulfur (S), nitrogen (N) or phosphorous (P). However, according to the present invention, the saturated and unsaturated hydrocarbons are preferably free from heteroatoms like oxygen (O), sulfur (S), nitrogen (N) and phosphorous (P).

As used herein, zeolite-based means that the catalyst comprises zeolite. The zeolite-based catalyst is preferably composed of ≥ 50 wt.%, more preferably of ≥ 60 wt.%, still more preferably of ≥ 70 wt.%, even more preferably of ≥ 80 wt.% and in particular preferably of ≥ 90 wt.% of zeolite; the weight percentages are based on the total weight of the zeolite-based catalyst. In a particular case, the zeolite-based catalyst consists of zeolite. As used herein, zeolite is given the same meaning as usual in the art. In particular, a zeolite has an aluminosilicate matrix with a tetrahedral arrangement of silicon (Si⁴⁺) and aluminium (Al³⁺) cations surrounded by four oxygen anions (O²⁻). This regularly results in a macromolecular three-dimensional structure of SiO₂ and AlO₂ tetrahedral building blocks. As the AlO₂ tetrahedral building blocks are negatively charged, zeolites regularly comprise additional charge-compensating cations, e.g., alkali metal cations, alkaline earth metal cations, protons and/or ammonia cations.

In step (ii) of the method for producing fuel according to the present invention, C6+ aromatics from step (i) are at least partially converted into C8+ aromatics by alkylation with unsaturated C₂-C₆ hydrocarbons from step (i) over an acid catalyst different from the zeolite of step (i). As used herein, C8+ aromatics are aromatics which contain 8 or more carbons (or carbon atoms). C8+ aromatics are valuable components of aviation fuels. Accordingly, the subsequent conversion of the C6+ aromatics of step (i) into C8+ aromatics can make the fuel produced by the inventive method particularly suitable for aviation fuel. As used herein, unsaturated C₂-C₆ hydrocarbons are hydrocarbons which contain 2, 3, 4, 5 or 6 carbons and have at least one unsaturation, especially one C-C double bond (olefins) or one C-C triple bond. Ethylene (C₂) and propylene (C₃) are olefins, are specific examples for such hydrocarbons and are particularly preferable comprised by the unsaturated C₂-C₆ hydrocarbons used in step (ii) for alkylating C6+ aromatics from step (i). By using unsaturated C₂-C₆ hydrocarbons from step (i) for the conversion of C6+ aromatics into C8+ aromatics no additional feed is needed and the method for producing fuel according to the present invention becomes at least partially self-supporting.

As used herein, an acid catalyst (or acidic catalyst) has Brønsted acidic sites, i.e., sites which function as proton donors and which are at least partially and preferably completely protonated. The Brønsted acidity of the acid catalyst used according to the present invention can for example be expressed in terms of the concentration of acidic cites, such that the acid catalyst has a concentration of Brønsted acidic sites of > 0 µmol/g, for example of > 1 µmol/g, as for example determined by pyridine adsorption (Py-adsorption), in particular IR-quantified Py-adsorption. It is preferred that the acid catalyst used in step (ii) is an acidic zeolite (which is however not the zeolite of step (i)).

In the method for producing fuel according to the present invention, the afore-listed catalysts are used, namely a metal oxide-based catalyst, a zeolite-based catalyst and an acid catalyst different from the zeolite-based catalyst. According to the present invention, all these catalysts are used in solid form. Here, solid form refers to the aggregation state of the respective catalysts, in particular under normal conditions of 298.15 K and 101.3 kPa.

The method for producing fuel according to the present invention uses CO₂ as a feedstock, which is at least partially converted and is hence not emitted to the environment. The inventive method can thus help to reduce the CO₂ footprint associated with the produced fuel. Herein, the CO₂ footprint refers to the amount of carbon dioxide released into the atmosphere.

The method for producing fuel according to the present invention can be carried out in one or more reaction vessels, especially reactors, which have previously been used for conversion of fossil feedstock. The inventive method may thus allow for existing infrastructure to be maintained.

The method for producing fuel according to the present invention combines ways of producing aromatics and larger hydrocarbons and yields a fuel comprising such aromatics and larger hydrocarbons in one single continuous reaction sequence. The inventive method can thereby lead to improved energy efficiency and/or improved cost efficiency of the fuel production. More specifically, the inventive method yields a fuel which comprises C8+ aromatics and C8+ hydrocarbons (wherein "C8+ hydrocarbons" refers to unsaturated C8+ hydrocarbons, saturated C8+ hydrocarbons or mixtures thereof). In other words, it is a method for producing fuel which comprises C8+ aromatics and C8+ hydrocarbons, which in some preferred cases includes at least partially and potentially completely hydrogenated C8+ aromatics and/or at least partially and potentially completely hydrogenated C8+ hydrocarbons. For example, unsaturated C8+ hydrocarbons produced in step (i) and C8+ aromatics produced in step (ii) are admixed in order to obtain the fuel which comprises C8+ aromatics and C8+ hydrocarbons. It may thus also be said that the method for producing fuel according to the present invention preferably comprises an additional step of mixing at least part of the unsaturated C8+ hydrocarbons produced in step (i) with at least part of the C8+ aromatics produced in step (ii). This additional step of mixing may occur after, or subsequently to, steps (i) and (ii). It is preferred that the additional mixing of C8+ aromatics and C8+ hydrocarbons occurs by simultaneously converting unsaturated C₂-C₆ hydrocarbons from step (i) into unsaturated C8+ hydrocarbons by oligomerisation and alkylating C6+ aromatics from step (i) with unsaturated C₂-C₆ hydrocarbons from step (i) into C8+ aromatics. Alternatively, the unsaturated C8+ hydrocarbons produced in step (i) may first at least partially be hydrogenated to saturated C8+ hydrocarbons as further described herein. Subsequently, the saturated C8+ hydrocarbons and the C8+ aromatics produced in step (ii) are admixed in order to produce a fuel which comprises C8+ aromatics and C8+ hydrocarbons. As a further alternative, the unsaturated C8+ hydrocarbons produced in step (i) may first be mixed with the C8+ aromatics and may thereafter at least partially be hydrogenated to saturated C8+ hydrocarbons as further described herein. In either case, the simultaneous production of C8+ aromatics and C8+ hydrocarbons through a cascade of specific reactions allows to utilize and ultimately valorise carbon dioxide as a raw material which is converted into a mixture which is particularly suitable for aviation fuel.

The method for producing fuel according to the present invention combines ways of producing aromatics and larger hydrocarbons and yields a fuel comprising such aromatics and larger hydrocarbons. The inventive method can thereby produce fuel which meets the requirements for aviation fuel.

The particular synthetic pathways of the conversion of methanol into C6+ aromatics in step (i) depend among others on the actually used zeolite-based catalyst and its structure. However, typical aromatics obtained in step (i) are benzene, toluene and xylene as well as mixtures thereof. The C6+ aromatics thus preferably comprise benzene, toluene and/or xylene. As used herein, xylene stands for any of o-xylene, m-xylene, p-xylene and mixtures thereof.

In the method for producing fuel according to the present invention, unsaturated C₂-C₆ hydrocarbons from step (i) are subsequently at least partially converted into unsaturated C8+ hydrocarbons by oligomerisation. Additionally, part of the unsaturated C₂-C₆ hydrocarbons from step (i) are further used in step (ii) for alkylating the C6+ aromatics from step (i) into C8+ aromatics. Hence, it may also be said that a first part of the unsaturated C₂-C₆ hydrocarbons from step (i) is subsequently converted into unsaturated C8+ hydrocarbons by oligomerisation, while a second part of the unsaturated C₂-C₆ hydrocarbons from step (i) is used in step (ii) for alkylating C6+ aromatics from step (i) at least partially into C8+ aromatics. Accordingly, the reference to "at least partially" in the context of the conversion of unsaturated C₂-C₆ hydrocarbons into unsaturated C8+ hydrocarbons by oligomerisation in step (i) means that a part of, but not all of, the unsaturated C₂-C₆ hydrocarbons are converted into unsaturated C8+ hydrocarbons as another part of the unsaturated C₂-C₆ hydrocarbons needs to be available for step (ii). Similarly, the reference to "at least partially" in the context of the alkylation of C6+ aromatics with unsaturated C₂-C₆ hydrocarbons into C8+ aromatics in step (ii) means that a part of, but not all of, the unsaturated C₂-C₆ hydrocarbons are used for converting C6+ aromatics into C8+ aromatics as another part of the unsaturated C₂-C₆ hydrocarbons needs to be available for step (i). As used herein, unsaturated C8+ hydrocarbons are hydrocarbons which contain 8 or more carbons (or carbon atoms). When unsaturated C₂-C₆ hydrocarbons from step (i) are oligomerised into unsaturated C8+ hydrocarbons, larger hydrocarbons can be produced by the inventive method for producing fuel. In this context, it is preferred that the oligomerisation of unsaturated C₂-C₆ hydrocarbons from step (i) and the alkylation of step (ii) are operated simultaneously in the same reactor. In this way, a simultaneous production of larger hydrocarbons and C8+ aromatics can be enabled in a cost-efficient manner. Further, the mixture which results from this preferred production may already be ready, or at least almost ready, for use in aviation applications. It is further preferred that the oligomerisation is performed at a temperature of 200 to 280°C. A temperature of at least 200°C can increase the efficiency of the oligomerisation and can thus improve the yield of desired larger hydrocarbons and the C8+ aromatics. At the same time, too high temperatures above 280°C may lead to a reduced energy efficiency and/or cost efficiency of the fuel production. It is also further preferred that the oligomerisation is performed at an absolute pressure of 4 to 8 MPa. A pressure of at least 4 MPa can also increase the efficiency of the oligomerisation and can thus also improve the yield of desired larger hydrocarbons and the C8+ aromatics. At the same time, too high pressures above 8 MPa may lead to a reduced energy efficiency and/or cost efficiency of the fuel production. In order to maximise the afore-mentioned effects, it is particularly preferred that the oligomerisation is performed at a temperature of 200 to 280°C and at an absolute pressure of 4 to 8 MPa.

For the method for producing fuel according to the present invention in which unsaturated C₂-C₆ hydrocarbons are subsequently at least partially converted into unsaturated C8+ hydrocarbons, the unsaturated C8+ hydrocarbons are subsequently at least partially hydrogenated with H₂ into saturated C8+ hydrocarbons (linear or branched C8+ hydrocarbons; as used herein, saturated C8+ hydrocarbons are saturated hydrocarbons which contain 8 or more carbons). Aviation fuel regularly needs higher amounts of saturated linear or branched hydrocarbons so that this preferred further conversion may render the fuel produced by the method according to the present invention even more suitable for aviation applications.

It is preferred that in a method for producing fuel according to the present invention, C8+ aromatics from step (ii) are at least partially hydrogenated with H₂ into saturated cyclic hydrocarbons (saturated cyclic C8+ hydrocarbons; or hydrogenated C8+ aromatics). Aviation fuel regularly needs higher amounts of saturated cyclic hydrocarbons so that this preferred further conversion may render the fuel produced by the method for producing fuel according to the present invention even more suitable for aviation applications.

For the preferred method for producing fuel according to the present invention in which an at least partial hydrogenation of unsaturated C8+ hydrocarbons and of C8+ aromatics occurs, it is further preferred that the at least partial hydrogenation of the unsaturated C8+ hydrocarbons and C8+ aromatics is operated simultaneously in the same reactor. In this way, the production of larger saturated liner and branched hydrocarbons as well as of saturated cyclic hydrocarbons can be simplified and hence made more cost efficient. Further, the mixture which results from this preferred production may already be ready, or at least almost ready, for use in aviation applications.

For the preferred method for producing fuel according to the present invention in which an at least partial hydrogenation of unsaturated C8+ hydrocarbons and of C8+ aromatics occurs, it is further preferred that the hydrogenation is performed at a temperature of 200 to 350°C. A temperature of at least 200°C can increase the efficiency of the hydrogenation and can thus improve the yield of the desired saturated hydrocarbons. At the same time, too high temperatures above 350°C may lead to a reduced energy efficiency and/or cost efficiency of the fuel production. It is also further preferred that the hydrogenation is performed at an absolute pressure of 2 to 6 MPa. A pressure of at least 2 MPa can also increase the efficiency of the hydrogenation and can thus also improve the yield of the desired saturated hydrocarbons. At the same time, too high pressures above 6 MPa may lead to a reduced energy efficiency and/or cost efficiency of the fuel production. In order to maximise the afore-mentioned effects, it is particularly preferred that the hydrogenation is performed at a temperature of 200 to 350°C and at an absolute pressure of 2 to 6 MPa.

It is preferred that in a method for producing fuel according to the present invention, step (i) additionally yields saturated hydrocarbons, having less than 8 carbons, which are subsequently at least partially converted in a steamcracker into unsaturated C₂-C₆ hydrocarbons and/or aromatics. Such shorter saturated hydrocarbons (C8- hydrocarbons, having seven or less carbon atoms) are typically less suitable for fuel and especially less suitable for aviation fuel. Their conversions into unsaturated C₂-C₆ hydrocarbons and/or aromatics allows to recycle them after leaving the steamcracker within the method for producing fuel according to the present invention itself. In this way, the shorter saturated hydrocarbons can be valorised by converting them ultimately into higher hydrocarbons which are typically suitable for fuel and especially suitable for aviation fuel.

It is preferred that in a method for producing fuel according to the present invention, the metal oxide-based catalyst in step (i) comprises In₂O₃/ZrO₂, ZnO/Cr₂O₃, ZnO/ZrO, Cu/ZnO/Al₂O₃, ZnAl₂O₃, Cr₂O₃ or a mixture thereof. The use of a metal oxide-based catalyst selected from In₂O₃/ZrO₂, ZnO/Cr₂O₃, ZnO/ZrO, Cu/ZnO/Al₂O₃, ZnAl₂O₃, Cr₂O₃ and mixtures thereof can lead to an increased conversion of CO₂ in step (i), which can further reduce the CO₂ footprint. Additionally, such a specifically selected metal oxide-based catalyst may already be used in existing infrastructure. Hence, such a metal oxide-based catalyst can help to maintain existing infrastructure.

It is preferred that in a method for producing fuel according to the present invention, the zeolite-based catalyst in step (i) comprises an MFI-type zeolite (especially a ZSM-5 zeolite or an HZSM-5 zeolite), a CHA-type zeolite, a BEA-type zeolite, an MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof. More preferably, the zeolite-based catalyst in step (i) comprises a ZSM-5 zeolite or an HZSM-5 zeolite, particularly preferable an HZSM-5 zeolite (an HZSM-5 zeolite is a proton-exchanged form of a ZSM-5-type zeolite). The listed zeolite types are indicated here by the codes attributed by the International Zeolite Association. The use of a zeolite-based catalyst which comprises a zeolite of the listed types can lead to an increased conversion of methanol into C6+ aromatics, which can further improve the energy efficiency and/or the cost efficiency of the fuel production. Additionally, such a zeolite-based catalyst of the listed types may already be used in existing infrastructure. Hence, such a zeolite-based catalyst of the listed types can help to maintain existing infrastructure. The mentioned effects can be particularly pronounced when the zeolite-based catalyst in step (i) comprises a ZSM-5 zeolite or an HZSM-5 zeolite, in particular an HZSM zeolite. Herein, a ZSM-5 zeolite and an HZSM-5 zeolite may be combinedly referred to as (H)ZSM-5 zeolite.

It is preferred that in a method for producing fuel according to the present invention, the zeolite-based catalyst in step (i) comprises a metal-modified zeolite. As used herein, "metal-modified" means that the zeolite contains metal cations different from alkali metal cations and alkaline earth metal cations. Preferred metal cations are Zn-cations, Ga-cations, Ag-cations, Mo-cations and/or Re-cations. Accordingly, it is particularly preferred that the zeolite-based catalyst in step (i) comprises a Zn-modified zeolite, a Ga-modified zeolite, an Ag-modified zeolite, an Mo-modified zeolite and/or a Re-modified zeolite. The use of such a metal-modified zeolite can lead to an even further increased conversion of methanol into aromatics, which can further improve the energy efficiency and/or the cost efficiency of the fuel production. Additionally, such a metal-modified zeolite may already be used in existing infrastructure. Hence, such a metal-modified zeolite can help to maintain existing infrastructure.

It is preferred that in a method for producing fuel according to the present invention, the metal oxide-based catalyst and the zeolite-based catalyst in step (i) are formulated in separate particles and are mixed equally over a catalyst bed. With such a catalyst bed made of separate particles comprising either metal oxide-based catalyst or zeolite-based catalyst, respectively, an increased conversion of CO₂ into methanol and subsequently of the methanol into C6+ aromatics may be achieved, which can further improve the energy efficiency and/or the cost efficiency of the fuel production.

It is preferred that in a method for producing fuel according to the present invention, the metal oxide-based catalyst and the zeolite-based catalyst in step (i) are formulated in separate particles and are staggered in separate layers throughout a catalyst bed, starting with the metal oxide-based catalyst at the top of the catalyst bed. With such staggered layers of separate particles comprising either metal oxide-based catalyst or zeolite-based catalyst, respectively, the arrangement of catalysts for step (i) can be simplified. Further, such staggered layers of separate particles comprising either metal oxide-based catalyst or zeolite-based catalyst, respectively, may already be used in existing infrastructure, thereby reducing the need for additional infrastructure.

It is preferred that in a method for producing fuel according to the present invention, the metal oxide-based catalyst and the zeolite-based catalyst in step (i) are formulated in the same particles. In this way, the formation of the catalyst mixture required for step (i) can be simplified which can help to reduce the costs for preparing the catalysts and may thus lower the overall costs of the method for producing fuel.

It is preferred that in a method for producing fuel according to the present invention, the acid catalyst in step (ii) comprises an acidic MFI-type zeolite (especially an HZSM-5 zeolite), an acidic CHA-type zeolite, an acidic BEA-type zeolite, an acidic MOR-type zeolite, an acidic FAU-type zeolite, an acidic MEL-type zeolite, an acidic FER-type zeolite, an acidic MTT-type zeolite, an acidic TON-type zeolite, an acidic ERI-type zeolite, an acidic MTW-type zeolite, an acidic MWW-type zeolite or a mixture thereof, provided the acid catalyst is different from the zeolite-based catalyst of step (i). More preferably, the acid catalyst in step (ii) comprises an HZSM-5 zeolite (which is a proton-exchanged form of a ZSM-5-type zeolite). Again, the listed zeolite types are indicated here by the codes attributed by the International Zeolite Association. The use of an acid catalyst which comprises a zeolite of the listed types can lead to an increased conversion of C6+ aromatics into C8+ aromatics, which can further improve the energy efficiency and/or the cost efficiency of the fuel production. Additionally, such an acid catalyst of the listed types may already be used in existing infrastructure. Hence, such an acid catalyst of the listed types can help to maintain existing infrastructure. The mentioned effects can be particularly pronounced when the zeolite-based catalyst in step (i) comprises an HZSM-5 zeolite.

It is preferred that in a method according to the present invention, step (i) is carried out at a temperature of 300 to 400°C. An increased temperature of at least 300°C in step (i) can lead to an increased conversion of the CO₂. This can help to further reduce the CO₂ footprint of the fuel produced by the inventive method. At the same time, too high temperatures above 400°C may lead to a reduced energy efficiency and/or cost efficiency of the fuel production. It is preferred that in a method according to the present invention, step (i) is performed at an absolute pressure of 4 to 8 MPa. An absolute pressure of at least 4 MPa in step (i) can lead to an increased conversion of the CO₂. This can help to further reduce the CO₂ footprint of the fuel produced by the inventive method. At the same time, too high pressures above 8 MPa may lead to a reduced energy efficiency and/or cost efficiency of the fuel production. In order to maximise the afore-mentioned effects, it is particularly preferred that step (i) is performed at a temperature of 300 to 400°C and at an absolute pressure of 4 to 8 MPa.

It is preferred that in a method for producing fuel according to the present invention, step (ii) is performed at a temperature of 200 to 280°C. A temperature of at least 200°C can increase the efficiency of the alkylation and can thus improve the yield of the desired C8+ aromatics. At the same time, too high temperatures above 280°C may lead to a reduced energy efficiency and/or cost efficiency of the fuel production. It is also further preferred that the alkylation in step (ii) is performed at an absolute pressure of 4 to 8 MPa. A pressure of at least 4 MPa can also increase the efficiency of the alkylation and can thus also improve the yield of the desired C8+ aromatics. At the same time, too high pressures above 8 MPa may lead to a reduced energy efficiency and/or cost efficiency of the fuel production. In order to maximise the afore-mentioned effects, it is particularly preferred that the alkylation is performed at a temperature of 200 to 280°C and at an absolute pressure of 4 to 8 MPa.

It is particularly preferred that in a method for producing fuel according to the present invention,
step (i) is carried out at a temperature of 300 to 400°C and under a pressure of 4 to 8 MPa, and
step (ii) is carried out at a temperature of 200 to 280°C and under a pressure of 4 to 8 MPa.

Such a preferred method for producing fuel according to the present invention can in particular help to reduce the CO₂ footprint associated with the produced fuel, may allow for existing infrastructure to be maintained, can lead to an improved energy efficiency and/or an improved cost efficiency of the fuel production and can produce fuel which meets the requirements for aviation fuel.

It is preferred that in a method for producing fuel according to the present invention, at least part of the H₂ of step (i) stems from a water electrolysis. In this way, the production of the used H₂ becomes more sustainable and can reduce the CO₂ footprint of the method for producing fuel according to the present invention. Similarly, for the preferred method for producing fuel according to the present invention in which an at least partial hydrogenation of unsaturated C8+ hydrocarbons and/or of C8+ aromatics occurs, it is also preferred that at least part of the H₂ used for such hydrogenation stems from a water electrolysis. Here, the production of the H₂ used in the hydrogenation becomes more sustainable and can further reduce the CO₂ footprint of the method for producing fuel according to the present invention.

A water electrolysis, or electrochemical water splitting, uses electricity to decompose water into oxygen and hydrogen gas. The electrolysis is regularly performed in an electrolytic cell (also referred to herein as an electrolyser) which comprises two electrodes, namely an anode and a cathode, between which an electric field is applied. Between the anode and the cathode, a solid oxide electroactive substrate is regularly sandwiched as an electrolyte. When operating the electrochemical cell, O²⁻ ions are removed from the water at the cathode to form hydrogen gas, and on the other hand electrons are removed from the O²⁻ ions at the anode to form oxygen gas. Accordingly, H₂ is produced at the cathode according to the following equation:

H₂O(*g*) → H₂(*g*) + O²⁻

At the same time, O₂ is produced at the anode according to the following equation:

2^{O2-} → O₂(*g*) + 4e⁻

The overall cell reaction is then as follows:

2 H₂O(*g*) → 2 H₂(*g*) + O₂(*g*)

It is preferred that in a method for producing fuel according to the present invention, the electrolyser operates at a temperature of 80 to 200°C. An increased temperature of at least 80°C can improve the production rate of H₂ and O₂. At the same time, too high temperatures above 200°C may lead to a reduced energy efficiency and/or cost efficiency of the fuel production. It is preferred that in a method for producing fuel according to the present invention, the electrolyser operates at an absolute pressure of ≥ 1 MPa. An absolute pressure of at least 1 MPa can improve the production rate of H₂ and O₂. In order to maximise the afore-mentioned effects, it is particularly preferred that the electrolyser operates at a temperature of 80 to 200°C and at an absolute pressure of ≥ 1 MPa.

It is further preferred that the water electrolysis occurs in an electrolyser which is electrically connected to a renewable energy source, wherein the renewable energy is more preferably selected from energy generated from sunlight, wind, rain, tides, waves and/or geothermal heat, more preferably generated from sunlight or wind. In this way, the production of the used H₂ and/or O₂ becomes even more sustainable and can further reduce the CO₂ footprint of the method for producing fuel according to the present invention.

The CO obtained in step (i) is at least partially recycled to the metal oxide-based catalyst as an additional feed in step (i). In this way, emissions of toxic CO to the atmosphere can be lowered and potentially completely avoided, thereby enhancing the safety of the method for producing fuel according to the present invention. At the same time, the carbon footprint of the fuel produced by the inventive method can be further reduced.

It is preferred that in a method for producing fuel according to the present invention, CO₂ fed in step (i) which remains unconverted is at least partially recycled to the metal oxide-based catalyst as an additional feed in step (i). In this way, emissions of CO₂ to the atmosphere can be lowered and potentially completely avoided, thereby further reducing the CO₂ footprint of the fuel produced by the inventive method.

It is preferred that in a method according to the present invention, the feed mixture in step (i) has a molar ratio of hydrogen to carbon oxides, i.e., a molar ratio H₂:COₓ, of 0.5 to 20, more preferably of 1 to 8, with x being 1 and/or 2, with x preferably being 2. With such a molar ratio of hydrogen to carbon oxides of 0.5 to 20 an increased conversion of CO₂ in step (i) may be achieved which can help to further reduce the CO₂ footprint of the fuel produced by the inventive method. These effects can be particularly pronounced when the ratio of hydrogen to carbon oxides is 1 to 8.

### Brief description of the drawings

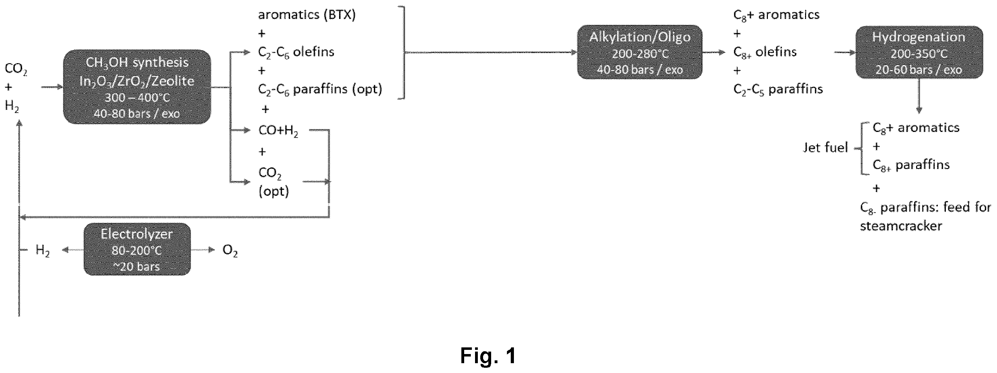

Fig. 1 shows an exemplary reaction flow chart for a method according to the present invention.

### Exemplary embodiment

The present invention is further described with reference to accompanying Fig. 1. In this exemplary embodiment, carbon dioxide (CO₂) and hydrogen (H₂) are fed to a first reaction zone in which a catalyst mixture made of the metal oxides In₂O₃ and ZrO₂ on the one hand and a zeolite on the other hand is present. Under conditions of the first reaction zone of a temperature of 300 to 400°C and an absolute pressure of 4 to 8 MPa (40 to 80 bars), the CO₂ and H₂ are firstly converted over the metal oxides into methanol (CH₃OH synthesis) and carbon monoxide (CO), wherein some unreacted CO₂ and H₂ may potentially also remain present. Further, under the same conditions the methanol is converted over the zeolite into C6+ aromatics, predominantly into benzene, toluene and xylene (BTX). Additional products of this first reaction stage are unsaturated C₂-C₆ hydrocarbons (here: C₂-C₆ olefins) and optionally (opt) saturated C₂-C₆ hydrocarbons (or C₂-C₆ paraffins). Carbon monoxide, potentially unreacted H₂ and potentially unreacted CO₂ are recycled as an additional feed to the first reaction zone. The overall reaction is exothermic and produces heat which can be used elsewhere.

Next, the produced C6+ aromatics and C₂-C₆ olefins are send to an alkylation/oligomerisation(oligo) reaction zone. Under conditions of the alkylation/oligomerisation(oligo) reaction zone of a temperature of 200 to 280°C and an absolute pressure of 4 to 8 MPa (40 to 80 bars) the C6+ aromatics are alkylated with C₂-C₆ olefins to yield C8+ aromatics, and some further C₂-C₆ olefins are oligomerised to yield C8+ olefins. The overall reaction is exothermic and produces heat which can be used elsewhere. The product mixture leaving the alkylation/oligomerisation(oligo) reaction zone further contains C₂₋₅ paraffins.

Next, the product mixture leaving the alkylation/oligomerisation(oligo) reaction zone is fed to a hydrogenation reaction zone to which additional H₂ is fed (not shown). Under conditions of the hydrogenation reaction zone of a temperature of 200 to 350°C and an absolute pressure of 2 to 6 MPa (20 to 60 bars) the C8+ aromatics are partially hydrogenated and the C8+ olefins are fully hydrogenated to give a product mixture containing C8+ aromatics and C8+ paraffins which together form an aviation fuel (jet fuel). The overall reaction is exothermic and produces heat which can be used elsewhere. The product mixture leaving the hydrogenation reaction zone further contains C₈₋ paraffins (having 7 or less carbons) which are sent to a steamcracker.

It is further seen from Fig. 1 that the hydrogen fed to the first reaction zone is produced by an electrolyser which electrolyses water (H₂O) and operates under conditions of a temperature of 80 to 200°C and an absolute pressure of about 2 MPa (20 bars).

## Claims

1. A method for producing fuel which comprises C8+ aromatics and saturated C8+ hydrocarbons, the method comprising the steps:
(i) converting a feed mixture comprising CO₂ with H₂ into a mixture comprising CO, C6+ aromatics and unsaturated C₂-C₆ hydrocarbons, wherein the CO₂ is at least partially converted into methanol using a metal oxide-based catalyst and wherein the methanol is at least partially converted into C6+ aromatics using a zeolite-based catalyst, wherein said unsaturated C₂-C₆ hydrocarbons are subsequently at least partially converted into unsaturated C8+ hydrocarbons by oligomerisation, wherein the unsaturated C8+ hydrocarbons are subsequently at least partially hydrogenated with H₂ to saturated C8+ hydrocarbons, and
(ii) alkylating C6+ aromatics from step (i) at least partially with unsaturated C₂-C₆ hydrocarbons from step (i) into C8+ aromatics using an acid catalyst different from the zeolite-based catalyst used in step (i),
wherein the CO obtained in step (i) is at least partially recycled to the metal oxide-based catalyst as an additional feed in step (i).

2. The method according to claim 1, wherein the C6+ aromatics comprise benzene, toluene and/or xylene.

3. The method according to any of the preceding claims, wherein C8+ aromatics from step (ii) are at least partially hydrogenated with H₂ into saturated cyclic hydrocarbons.

4. The method according to any of the preceding claims, wherein step (i) additionally yields saturated C8- hydrocarbons which are subsequently at least partially converted in a steamcracker into unsaturated C₂-C₆ hydrocarbons and/or aromatics.

5. The method according to any of the preceding claims, wherein the metal oxide-based catalyst in step (i) comprises In₂O₃/ZrO₂, ZnO/Cr₂O₃, ZnO/ZrO, Cu/ZnO/Al₂O₃, ZnAl₂O₃, Cr₂O₃ or a mixture thereof.

6. The method according to any of the preceding claims, wherein the zeolite-based catalyst in step (i) comprises an MFI-type zeolite, a CHA-type zeolite, a BEA-type zeolite, an MOR-type zeolite, an FAU-type zeolite, an MEL-type zeolite, an FER-type zeolite, an MTT-type zeolite, a TON-type zeolite, an ERI-type zeolite, an MTW-type zeolite, an MWW-type zeolite or a mixture thereof.

7. The method according to any of the preceding claims, wherein step (i) is carried out at a temperature of 300 to 400°C and/or under a pressure of 4 to 8 MPa.

8. The method according to any of the preceding claims, wherein step (ii) is carried out at a temperature of 200 to 280°C and/or under a pressure of 4 to 8 MPa.

9. The method according to any of the preceding claims, wherein at least part of the H₂ of step (i) stems from a water electrolysis.

10. The method according to any of the preceding claims, wherein CO₂ fed in step (i) which remains unconverted is at least partially recycled to the metal oxide-based catalyst as an additional feed in step (i).

11. The method according to any of the preceding claims wherein following said method the thereby obtained fuel is used as aviation fuel.

## Patentansprüche

1. Verfahren zur Herstellung eines Brennstoffs, der C8+-Aromaten und gesättigte C8+-Kohlenwasserstoffe enthält, wobei das Verfahren die folgenden Schritte umfasst:
(i) Umwandlung eines Einsatzgemisches, das CO₂ und H₂ enthält, in ein Gemisch, das CO, C6+-Aromaten und ungesättigte C2-C6-Kohlenwasserstoffe enthält, wobei das CO₂ unter Verwendung eines Katalysators auf Metalloxidbasis zumindest teilweise in Methanol umgewandelt wird und wobei das Methanol unter Verwendung eines Katalysators auf Zeolithbasis zumindest teilweise in C6+ Aromaten unter Verwendung eines Katalysators auf Zeolithbasis umgewandelt wird, wobei die ungesättigten C2-C6-Kohlenwasserstoffe anschließend durch Oligomerisierung zumindest teilweise in ungesättigte C8+-Kohlenwasserstoffe umgewandelt werden, wobei die ungesättigten C8+-Kohlenwasserstoffe anschließend mit H2 zumindest teilweise zu gesättigten C8+-Kohlenwasserstoffen hydriert werden, und
(ii) Alkylierung von C6+-Aromaten aus Schritt (i) zumindest teilweise mit ungesättigten C2-C6-Kohlenwasserstoffen aus Schritt (i) zu C8+-Aromaten unter Verwendung eines Säurekatalysators, der sich von dem in Schritt (i) verwendeten Katalysator auf Zeolithbasis unterscheidet,
wobei das in Schritt (i) erhaltene CO zumindest teilweise zu dem auf Metalloxid basierenden Katalysator als zusätzlicher Einsatzstoff in Schritt (i) zurückgeführt wird.

2. Verfahren nach Anspruch 1, wobei die C6+-Aromaten Benzol, Toluol und/oder Xylol umfassen.

3. Verfahren nach einem der vorstehenden Ansprüche, wobei die C8+-Aromaten aus Schritt (ii) zumindest teilweise mit H₂ zu gesättigten zyklischen Kohlenwasserstoffen hydriert werden.

4. Verfahren nach einem der vorstehenden Ansprüche, wobei Schritt (i) zusätzlich gesättigte C8-Kohlenwasserstoffe liefert, die anschließend zumindest teilweise in einem Dampfcracker zu ungesättigten C2-C6-Kohlenwasserstoffen und/oder Aromaten umgesetzt werden.

5. Verfahren nach einem der vorstehenden Ansprüche, wobei der in Schritt (i) verwendete auf Metalloxid basierende Katalysator In₂O₃/ZrO₂, ZnO/Cr₂O₃, ZnO/ZrO, Cu/ZnO/Al₂O₃, ZnAl₂O₄, Cr₂O₃ oder eine Mischung davon umfasst.

6. Verfahren nach einem der vorstehenden Ansprüche, wobei der in Schritt (i) verwendete zeolithbasierte Katalysator einen Zeolith vom MFI-Typ, einen Zeolith vom CHA-Typ, einen Zeolith vom BEA-Typ, einen Zeolith vom MOR-Typ, einen Zeolith vom FAU-Typ, einen Zeolith vom MEL-Typ, einen Zeolith vom FER-Typ, einen Zeolith vom MTT-Typ, einen Zeolith vom TON-Typ, einen Zeolith vom ERI-Typ, einen Zeolith vom MTW-Typ, einen Zeolith vom MWW-Typ oder eine Mischung davon umfasst.

7. Verfahren nach einem der vorstehenden Ansprüche, wobei Schritt (i) bei einer Temperatur von 300 bis 400 °C und/oder unter einem Druck von 4 bis 8 MPa durchgeführt wird.

8. Verfahren nach einem der vorstehenden Ansprüche, wobei Schritt (ii) bei einer Temperatur von 200 bis 280 °C und/oder unter einem Druck von 4 bis 8 MPa durchgeführt wird.

9. Verfahren nach einem der vorstehenden Ansprüche, wobei zumindest ein Teil des in Schritt (i) verwendeten H₂ aus einer Wasserelektrolyse stammt.

10. Verfahren nach einem der vorstehenden Ansprüche, wobei das in Schritt (i) zugeführte CO₂, das nicht umgewandelt wurde, zumindest teilweise zu dem auf Metalloxid basierenden Katalysator als zusätzlicher Einsatzstoff in Schritt (i) zurückgeführt wird.

11. Verfahren nach einem der vorstehenden Ansprüche, wobei der nach diesem Verfahren erhaltene Brennstoff als Flugkraftstoff verwendet wird.

## Revendications

1. Un procédé de production de carburant qui comprend des hydrocarbures aromatiques C8+ et des hydrocarbures saturés C8+, le procédé comprenant les étapes :
(i) conversion d'un mélange d'alimentation comprenant du CO₂ avec de H₂ en un mélange comprenant du CO, des hydrocarbures aromatiques C6+ et des hydrocarbures insaturés C₂-C₆, dans lequel le CO₂ est au moins partiellement converti en méthanol à l'aide d'un catalyseur à base d'oxyde de métaux et dans lequel le méthanol est au moins partiellement converti en aromatiques C6+ à l'aide d'un catalyseur à base de zéolithe, dans lequel lesdits hydrocarbures insaturés C₂-C₆ sont ensuite au moins partiellement convertis en hydrocarbures insaturés C8+ par oligomérisation, dans lequel les hydrocarbures insaturés C8+ sont ensuite au moins partiellement hydrogénés avec du H₂ en hydrocarbures saturés C8+, et
(ii) alkylation des aromatiques C6+ de l'étape (i) au moins partiellement avec des hydrocarbures insaturés C₂-C₆ de l'étape (i) en aromatiques C8+ à l'aide d'un catalyseur acide différent du catalyseur à base de zéolithe utilisé dans l'étape (i),
dans lequel le CO obtenu à l'étape (i) est au moins partiellement recyclé vers le catalyseur à base d'oxyde de métaux en tant qu'alimentation supplémentaire à l'étape (i).

2. Procédé selon la revendication 1, dans lequel les aromatiques C6+ comprennent le benzène, le toluène et/ou le xylène.

3. Procédé selon l'une quelconque des revendications précédentes, dans lequel les aromatiques C8+ de l'étape (ii) sont au moins partiellement hydrogénés avec du H₂ en hydrocarbures saturés cycliques.

4. Procédé selon l'une quelconque des revendications précédentes, dans lequel l'étape (i) donne en outre des hydrocarbures saturés C8- qui sont ensuite au moins partiellement convertis dans un vapocraqueur en hydrocarbures insaturés C₂-C₆ et/ou en aromatiques.

5. Procédé selon l'une quelconque des revendications précédentes, dans lequel le catalyseur à base d'oxyde de métaux dans l'étape (i) comprend In₂O₃/ZrO₂, ZnO/Cr₂O₃, ZnO/ZrO, Cu/ZnO/Al₂O₃, ZnAl₂O₃, Cr₂O₃ ou un mélange de ceux-ci.

6. Procédé selon l'une quelconque des revendications précédentes, dans lequel le catalyseur à base de zéolithe dans l'étape (i) comprend une zéolithe de type MFI, une zéolithe de type CHA, une zéolithe de type BEA, une zéolithe de type MOR, une zéolithe de type FAU, une zéolithe de type MEL, une zéolithe de type FER, une zéolithe de type MTT, une zéolithe de type TON, une zéolithe de type ERI, une zéolithe de type MTW, une zéolithe de type MWW ou un mélange de celles-ci.

7. Procédé selon l'une quelconque des revendications précédentes, dans lequel l'étape (i) est réalisée à une température de 300 à 400 °C et/ou sous une pression de 4 à 8 MPa.

8. Procédé selon l'une quelconque des revendications précédentes, dans lequel l'étape (ii) est réalisée à une température de 200 à 280 °C et/ou sous une pression de 4 à 8 MPa.

9. Procédé selon l'une quelconque des revendications précédentes, dans lequel au moins une partie du H₂ de l'étape (i) provient d'une électrolyse de l'eau.

10. Procédé selon l'une quelconque des revendications précédentes, dans lequel le CO₂ introduit dans l'étape (i) qui reste non converti est au moins partiellement recyclé vers le catalyseur à base d'oxyde de métaux en tant qu'alimentation supplémentaire dans l'étape (i).

11. Procédé selon l'une quelconque des revendications précédentes, dans lequel, après ledit procédé, le carburant ainsi obtenu est utilisé comme carburant pour l'aviation.
